# EUROPEAN PATENT APPLICATION

(11) **EP 4 599 834 A1**
(43) Date of publication of application: **13.08.2025**
(21) Application number: 23874803.2
(22) Date of filing: 02.10.2023
(51) Int. Cl.: A61K 35/32, A61K 9/08, A61K 35/28, A61P 9/00, A61P 25/00, A61P 43/00, C12N 5/074, C12N 5/0793

(54) **AGENT FOR TREATING OR PREVENTING CEREBRAL PALSY**

(30) Priority: 03.10.2022 JP 2022159434; 03.10.2022 WO PCT/JP2022/036892; 25.04.2023 JP 2023071438
(71) Applicant: Kidswell Bio Corporation, Tokyo 104-0033 (JP)
(72) Inventor: SATO Yoshiaki, Nagoya-shi, Aichi 464-8601 (JP); KANZAWA Takahiro, Nagoya-shi, Aichi 464-8601 (JP); SHIMIZU Shinobu, Nagoya-shi, Aichi 464-8601 (JP); HAYAKAWA Masahiro, Nagoya-shi, Aichi 464-8601 (JP); YUKAWA Hiroshi, Nagoya-shi, Aichi 464-8601 (JP); MITANI Yasuyuki, Tokyo 104-0033 (JP); ARIKI Hiromi, Sapporo-shi, Hokkaido 001-0021 (JP); FUKUDA Noritaka, Sapporo-shi, Hokkaido 001-0021 (JP)
(74) Representative: Hoffmann Eitle
(86) International application number: PCT/JP2023/035859
(87) International publication number: WO 2024/075676

(57) **Abstract**

The present invention relates to an agent for preventing or treating cerebral palsy, comprising dental pulp stem cells, wherein the prevention or treatment agent is administered to a subject with symptoms of cerebral palsy.

## Description

### [Technical Field]

### Related application:

The present specification incorporates the contents described in the specifications of Japanese Patent Application No. 2022-159434 (filed on October 3, 2022), PCT/JP2022/036892 (filed on October 3, 2022), and Japanese Patent Application No. 2023-071438(filed on April 25, 2023) on which the priority of the present application is based.

### Technical Field:

The present invention relates to an agent for preventing or treating cerebral palsy, etc.

### [Background Art]

Cerebral palsy is caused by various perinatal cerebral damages including neonatal hypoxic-ischemic encephalopathy (HIE) and leads to dyskinesia or neurological symptoms such as epilepsy at the chronic stage (chronic phase). There is a need for the development of a treatment method that is effective even after symptoms of cerebral palsy are confirmed at the chronic stage.

Non Patent Literature 1 has reported that symptoms of cerebral palsy are improved as a result of administering mesenchymal stem cells derived from umbilical cord blood to the subarachnoidal spaces of 4-to 14-year-old cerebral palsy patients. However, there are still few cases where umbilical cord blood is preserved at birth. Particularly, it is difficult to use mesenchymal stem cells derived from autologous umbilical cord blood (autologous transplantation) at the chronic stage. Stem cells, also including diverse stem cells other than the stem cells derived from umbilical cord blood, differ largely in the ability to differentiate, secretory factors, and the like, depending on their origins. Therefore, the type of stem cells having an improving effect on cerebral palsy symptoms cannot be predicted.

### [Citation List]

### [Non Patent Literature]

[Non Patent Literature 1] Amanat et al., Stem Cell Research & Therapy (2021) 12: 439

### [Summary of Invention]

### [Technical Problem]

An object of the present invention is to provide an agent for preventing or treating cerebral palsy.

### [Solution to Problem]

The present inventors have pursued diligent studies in light of the object and consequently found that, surprisingly, dental pulp stem cells have a prophylactic or therapeutic effect on cerebral palsy and a promoting effect on neurogenesis in the brain. The present inventors have pursued further studies based on this finding and consequently completed the present invention. Specifically, the present invention encompasses the following aspects.

According to the first aspect, the present invention relates to the following [1] to [20].
[1] An agent for preventing or treating cerebral palsy, comprising dental pulp stem cells, wherein the prevention or treatment agent is administered to a subject with symptoms of cerebral palsy.
[2] The prevention or treatment agent according to [1], wherein the cerebral palsy is chronic stage cerebral palsy.
[3] The prevention or treatment agent according to [2], wherein the chronic stage is from infancy onward.
[4] The prevention or treatment agent according to any of [1] to [3], wherein the prevention or treatment agent is administered in multiple doses (2 to 20 doses, preferably 2 to 10 doses, further preferably 2 to 5 doses).
[5] The prevention or treatment agent according to any of [1] to [4], wherein the dental pulp stem cells are human deciduous dental pulp stem cells (stem cells derived from human deciduous dental pulp).
[6] The prevention or treatment agent according to any of [1] to [5], wherein the cerebral palsy is cerebral palsy caused by hypoxia and/or ischemia.
[7] The prevention or treatment agent according to any of [1] to [6], wherein the cerebral palsy is caused by perinatal cerebral damage.
[8] The prevention or treatment agent according to any of [1] to [7], wherein the prevention or treatment agent is used for improving a motor function and/or a memory learning function of the subject with symptoms of cerebral palsy.
[9] The prevention or treatment agent according to any of [1] to [8], wherein the subject is a human.
[10] The prevention or treatment agent according to any of [1] to [9], wherein the prevention or treatment agent causes neurogenesis in the brain of the subject.
[11] The prevention or treatment agent according to any of [1] to [10], wherein the dental pulp stem cells consist of a stem cell population derived from human deciduous dental pulp, the stem cell population being characterized in that 90% or more of the stem cell population is CD117-negative, CD73-positive, CD90-positive, and CD105-positive.
[12] The prevention or treatment agent according to [11], wherein 90% or more of the stem cell population is CD325-negative or is CD51-positive.
[13] The prevention or treatment agent according to [11] or [12], wherein the stem cell population has at least one of the following characteristics 1) to 3):
   1) producing SCF (stem cell factor) at a weight ratio of 1/10 or more to relative IL-6,
   2) producing ANGPTL4 (angiopoietin-like 4) at a weight ratio of 5 or more relative to IL-6,
   3) producing BIGH3 (transforming growth factor-beta-induced) at a weight ratio of 450 or more relative to IL-6.
[14] The prevention or treatment agent according to any of [11] to [13], wherein the stem cell population has at least one of the following characteristics 1) to 3):
   1) producing at least 0.1 ng of SCF (stem cell factor) in 48 hours per 1 × 10⁶ cells,
   2) producing at least 500 ng of BIGH3 (transforming growth factor-beta-induced) in 48 hours per 1 × 10⁶ cells,
   3) producing at least 5 ng of ANGPTL4 in 48 hours per 1 × 10⁶ cells.
[15] The prevention or treatment agent according to any of [11] to [14], wherein the stem cell population is obtained by culturing cells enzymatically isolated from the human dental pulp in a medium free of FBS (fetal bovine serum) in the presence of a human platelet lysate.
[16] The prevention or treatment agent according to any of [1] to [15], wherein the prevention or treatment agent comprises the dental pulp stem cells as an active ingredient (preferably, a single active ingredient).
[17] The prevention or treatment agent according to any of [1] to [16], wherein the dental pulp stem cells are human deciduous dental pulp stem cells not induced to differentiate and/or non-transgenic human deciduous dental pulp stem cells.
[18] The prevention or treatment agent according to any of [1] to [17], wherein the dental pulp stem cells are human deciduous dental pulp stem cells not induced to differentiate into neurons.
[19] The prevention or treatment agent according to any of [1] to [18], wherein the subject is a human who has undergone neonatal asphyxia and/or been diagnosed with periventricular leukomalacia.
[20] The treatment agent according to any of [1] to [19], wherein the subject is a human of 6 months or later after birth.

According to the second aspect, the present invention relates to the following [1] to [15].
[1] A composition for use in the prevention or treatment of cerebral palsy, comprising dental pulp stem cells, wherein the composition is administered to a subject with symptoms of cerebral palsy.
[2] The composition for use according to [1], wherein the cerebral palsy is chronic stage cerebral palsy.
[3] The composition for use according to [2], wherein the chronic stage is from infancy onward.
[4] The composition for use according to any of [1] to [3], wherein the composition is administered in multiple doses (2 to 20 doses, preferably 2 to 10 doses, further preferably 2 to 5 doses) to the subject.
[5] The composition for use according to any of [1] to [4], wherein the dental pulp stem cells are human deciduous dental pulp stem cells (stem cells derived from human deciduous dental pulp), preferably human deciduous dental pulp stem cells not induced to differentiate (particularly, not induced to differentiate into neurons) and/or non-transgenic human deciduous dental pulp stem cells.
[6] The composition for use according to any of [1] to [5], wherein the cerebral palsy is cerebral palsy caused by hypoxia and/or ischemia.
[7] The composition for use according to any of [1] to [6], wherein the cerebral palsy is caused by perinatal cerebral damage.
[8] The composition for use according to any of [1] to [7], wherein the composition is used for improving a motor function and/or a memory learning function of the subject with symptoms of cerebral palsy.
[9] The composition for use according to any of [1] to [8], wherein the subject is a human, for example, a human of 6 months or later after birth or a human who has undergone neonatal asphyxia and/or been diagnosed with periventricular leukomalacia.
[10] The composition for use according to any of [1] to [9], wherein the composition causes neurogenesis in the brain of the subject.
[11] The composition for use according to any of [1] to [10], wherein the dental pulp stem cells consist of a stem cell population derived from human deciduous dental pulp, the stem cell population being characterized in that 90% or more of the stem cell population is CD117-negative, CD73-positive, CD90-positive, and CD105-positive.
[12] The composition for use according to [11], wherein 90% or more of the stem cell population is CD325-negative or is CD51-positive.
[13] The composition for use according to [11] or [12], wherein the stem cell population has at least one of the following characteristics 1) to 3):
   1) producing SCF (stem cell factor) at a weight ratio of 1/10 or more relative to IL-6,
   2) producing ANGPTL4 (angiopoietin-like 4) at a weight ratio of 5 or more relative to IL-6,
   3) producing BIGH3 (transforming growth factor-beta-induced) at a weight ratio of 450 or more relative to IL-6.
[14] The composition for use according to any of [11] to [13], wherein the stem cell population has at least one of the following characteristics 1) to 3):
   1) producing at least 0.1 ng of SCF (stem cell factor) in 48 hours per 1 × 10⁶ cells,
   2) producing at least 500 ng of BIGH3 (transforming growth factor-beta-induced) in 48 hours per 1 × 10⁶ cells,
   3) producing at least 5 ng of ANGPTL4 in 48 hours per 1 × 10⁶ cells.
[15] The composition for use according to any of [11] to [14], wherein the stem cell population is obtained by culturing cells enzymatically isolated from the human dental pulp in a medium free of FBS (fetal bovine serum) in the presence of a human platelet lysate.

According to the third aspect, the present invention relates to the following [1] to [20].
[1] A method for preventing or treating cerebral palsy, comprising administering dental pulp stem cells to a subject, wherein the subject is a subject with symptoms of cerebral palsy.
[2] The method according to [1], wherein the cerebral palsy is chronic stage cerebral palsy.
[3] The method according to [2], wherein the chronic stage is from infancy onward.
[4] The method according to [1], wherein the dental pulp stem cells are administered in multiple doses (2 to 20 doses, preferably 2 to 10 doses, further preferably 2 to 5 doses).
[5] The method according to [1], wherein the dental pulp stem cells are human deciduous dental pulp stem cells (stem cells derived from human deciduous dental pulp).
[6] The method according to [1], wherein the cerebral palsy is cerebral palsy caused by hypoxia and/or ischemia.
[7] The method according to [1], wherein the cerebral palsy is caused by perinatal cerebral damage.
[8] The method according to [1], wherein the dental pulp stem cells improve a motor function and/or a memory learning function of the subject with symptoms of cerebral palsy.
[9] The method according to [1], wherein the subject is a human.
[10] The method according to [1], wherein the dental pulp stem cells cause neurogenesis in the brain of the subject.
[11] The method according to [1], wherein the dental pulp stem cells consist of a stem cell population derived from human deciduous dental pulp, the stem cell population being characterized in that 90% or more of the stem cell population is CD117-negative, CD73-positive, CD90-positive, and CD105-positive.
[12] The method according to [11], wherein 90% or more of the stem cell population is CD325-negative or is CD51-positive.
[13] The method according to [11], wherein the stem cell population has at least one of the following characteristics 1) to 3):
   1) producing SCF (stem cell factor) at a weight ratio of 1/10 or more relative to IL-6,
   2) producing ANGPTL4 (angiopoietin-like 4) at a weight ratio of 5 or more relative to IL-6,
   3) producing BIGH3 (transforming growth factor-beta-induced) at a weight ratio of 450 or more relative to IL-6.
[14] The method according to [11], wherein the stem cell population has at least one of the following characteristics 1) to 3):
   1) producing at least 0.1 ng of SCF (stem cell factor) in 48 hours per 1 × 10⁶ cells,
   2) producing at least 500 ng of BIGH3 (transforming growth factor-beta-induced) in 48 hours per 1 × 10⁶ cells,
   3) producing at least 5 ng of ANGPTL4 in 48 hours per 1 × 10⁶ cells.
[15] The method according to [11], wherein the stem cell population is obtained by culturing cells enzymatically isolated from the human dental pulp in a medium free of FBS (fetal bovine serum) in the presence of a human platelet lysate.
[16] The method according to [1], wherein the dental pulp stem cells are administered as a single active ingredient.
[17] The method according to [1], wherein the dental pulp stem cells are human deciduous dental pulp stem cells not induced to differentiate and/or non-transgenic human deciduous dental pulp stem cells.
[18] The method according to [1], wherein the dental pulp stem cells are human deciduous dental pulp stem cells not induced to differentiate into neurons.
[19] The method according to [1], wherein the subject is a human who has undergone neonatal asphyxia and/or been diagnosed with periventricular leukomalacia.
[20] The method according to [1], wherein the subject is a human of 6 months or later after birth.

According to the fourth aspect, the present invention relates to the following [1] to [13].
[1] Use of dental pulp stem cells in the production of an agent for preventing or treating cerebral palsy, wherein the prevention or treatment agent is for a subject with symptoms of cerebral palsy.
[2] The use according to [1], wherein the cerebral palsy is chronic stage cerebral palsy.
[3] The use according to [2], wherein the chronic stage is from infancy onward.
[4] The use according to any of [1] to [3], wherein the dental pulp stem cells are human deciduous dental pulp stem cells (stem cells derived from human deciduous dental pulp).
[5] The use according to any of [1] to [4], wherein the cerebral palsy is cerebral palsy caused by hypoxia and/or ischemia.
[6] The use according to any of [1] to [5], wherein the cerebral palsy is caused by perinatal cerebral damage.
[7] The use according to any of [1] to [6], wherein the prevention or treatment agent is for improving a motor function and/or a memory learning function of the subject with symptoms of cerebral palsy.
[8] The use according to any of [1] to [7], wherein the subject is a human.
[9] The use according to any of [1] to [8], wherein the dental pulp stem cells consist of a stem cell population derived from human deciduous dental pulp, the stem cell population being characterized in that 90% or more of the stem cell population is CD117-negative, CD73-positive, CD90-positive, and CD105-positive.
[10] The use according to [9], wherein 90% or more of the stem cell population is CD325-negative or is CD51-positive.
[11] The use according to [9] or [10], wherein the stem cell population has at least one of the following characteristics 1) to 3):
   1) producing SCF (stem cell factor) at a weight ratio of 1/10 or more relative to IL-6,
   2) producing ANGPTL4 (angiopoietin-like 4) at a weight ratio of 5 or more relative to IL-6,
   3) producing BIGH3 (transforming growth factor-beta-induced) at a weight ratio of 450 or more relative to IL-6.
[12] The use according to any of [9] to [11], wherein the stem cell population has at least one of the following characteristics 1) to 3):
   1) producing at least 0.1 ng of SCF (stem cell factor) in 48 hours per 1 × 10⁶ cells,
   2) producing at least 500 ng of BIGH3 (transforming growth factor-beta-induced) in 48 hours per 1 × 10⁶ cells,
   3) producing at least 5 ng of ANGPTL4 in 48 hours per 1 × 10⁶ cells.
[13] The use according to any of [9] to [12], wherein the stem cell population is obtained by culturing cells enzymatically isolated from the human dental pulp in a medium free of FBS (fetal bovine serum) in the presence of a human platelet lysate.

### [Advantageous Effect of Invention]

The present invention can provide an agent for preventing or treating cerebral palsy using dental pulp stem cells.

### [Brief Description of Drawings]

[Figure 1] Figure 1 shows results of a horizontal ladder test in the case of single-dose administration of cells (Test Example 2).
[Figure 2] Figure 2 shows results of a horizontal ladder test in the case of multiple-dose administration of cells (Test Example 2).
[Figure 3] Figure 3 shows a diagram in which the results of the horizontal ladder test (Test Example 2) were compared using the single-dose administration of cells and the multiple-dose administration of cells.
[Figure 4] Figure 4 shows results of a cylinder test in the case of multiple-dose administration of cells (Test Example 2).
[Figure 5] Figure 5 shows results of shuttle avoidance in the case of multiple-dose administration of cells (Test Example 2).
[Figure 6] Figure 6 shows results of measuring a body weight (Test Example 3).
[Figure 7] Figure 7 shows results of measuring a brain weight ratio (Test Example 3).
[Figure 8] Figure 8 shows a fluorescence intensity ratio of deciduous dental pulp stem cells after administration of the cells (which indicates the abundance of the cells).
[Figure 9] Figure 9 shows the ratio of BrdU/DCX double stain-positive cells in a hippocampal dentate gyrus lesion of the brain after administration of the cells.
[Figure 10] Figure 10 shows the number of BrdU-positive cells in a hippocampal dentate gyrus lesion of the brain after administration of the cells.
[Figure 11] Figure 11 shows the number of NeuN-positive cells in a hippocampal dentate gyrus lesion of the brain after administration of the cells.
[Figure 12] Figure 12 shows results of a rotarod test of a perinatal hypoxic-ischemic encephalopathy model in the case of single-dose administration of cells (Test Example 7).
[Figure 13] Figure 13 shows results of a cylinder test of a severe perinatal hypoxic-ischemic encephalopathy model in the case of multiple-dose administration of cells (Test Example 8).
[Figure 14] Figure 14 shows a neural stem cell proliferation rate by the coculture of neural stem cells and deciduous dental pulp stem cells, bone marrow-derived mesenchymal stem cells, or fibroblasts (Test Example 11).
[Figure 15] Figure 15 shows the amount of BDNF in a culture supernatant by coculture with neural stem cells (Test Example 11).

### [Description of Embodiments]

The terms "contain (containing)" and "comprise (comprising)" as used herein conceptually include the terms "contain (containing)", "comprise (comprising)", "consist (consisting) essentially of", and " consist (consisting) only of".

In one aspect, the present invention relates to an agent for preventing or treating cerebral palsy, comprising dental pulp stem cells. In another aspect, the present invention relates to an agent for promoting neurogenesis in the brain of a subject with cerebral palsy symptoms, comprising dental pulp stem cells (in the present specification, these agents are also collectively referred to as the "prevention or treatment agent of the present invention"). Hereinafter, these agents will be described.

### 1. Dental pulp stem cell

### 1.1 Origin

The dental pulp stem cells are not particularly limited as long as the stem cells are obtained from dental pulp (derived from dental pulp). Examples of the dental pulp stem cells include deciduous dental pulp stem cells (dental pulp stem cells derived from deciduous teeth) and permanent dental pulp stem cells (dental pulp stem cells derived from permanent teeth). The dental pulp stem cells are stem cells derived from low invasively and easily collectable neural crest and are a cell population coexpressing various undifferentiated nervous system markers and mesenchymal stem cell markers (Miura M. et al; Proc Natl Acad Sci U S A. 2003 May 13; 100 (10): 5807-123). The dental pulp stem cells are preferably deciduous dental pulp stem cells from the viewpoint of a prophylactic or therapeutic effect on cerebral palsy, etc., particularly preferably deciduous dental pulp stem cells collected from a deciduous tooth within 48 hours after fallout or tooth extraction.

Examples of the organism from which the dental pulp stem cells are derived include, but are not particularly limited to, various mammals such as humans, monkeys, mice, rats, dogs, cats, rabbits, pigs, horses, bovines, sheep, goats, and deers. The dental pulp stem cells are preferably human dental pulp stem cells, further preferably human deciduous dental pulp stem cells, particularly preferably deciduous dental pulp stem cells obtained from a deciduous tooth within 48 hours after fallout or tooth extraction.

In relation to a subject (patient, etc.) to which the prevention or treatment agent of the present invention is applied, the dental pulp stem cells are preferably dental pulp stem cells of the same organism species there as (i.e., derived from a human if the subject is a human) in order to suppress or circumvent rejection. The dental pulp stem cells can be autologous dental pulp stem cells or allogeneic dental pulp stem cells. The "prevention" includes a state in which the onset of symptoms or decline in physical function is suppressed or slowed down. The "treatment" includes the alleviation of symptoms or improvement in declined physical function. Examples of such symptoms or functions include motor functions, posture, memory, and learning for cerebral palsy.

The dental pulp stem cells can be selected as adherent cells among dental pulp cells. Adherent cells among dental pulp cells collected from a deciduous tooth that has fallen out or a permanent tooth, or passaged cells thereof can be used as the "dental pulp stem cells". The dental pulp stem cells can be prepared by, for example, the following method described in Japanese Patent Laid-Open No. 2011-219432.

### (1) Collection of dental pulp

A deciduous tooth that has fallen out spontaneously (or an extracted deciduous tooth or permanent tooth) is disinfected with a chlorhexidine or povidone-iodine solution. Then, the crown of the tooth is divided, and a dental pulp tissue is recovered using a dental reamer.

### (2) Enzymatic treatment

The collected dental pulp tissue was suspended in a basal medium (e.g., MEMα) and treated with 0.02 to 2 mg/ml tissue-degrading enzyme (e.g., collagenase or dispase). The dental pulp cells thus enzymatically treated are recovered by centrifugal operation.

### (3) Cell culture (selection of adherent cell)

The cells are resuspended in a basal medium and inoculated to a dish for adhesion cell culture. The cells are cultured in an incubator adjusted to 5% CO₂ and 37°C and then washed, if necessary, and adherent cells that have formed colonies are then treated with 0.05% trypsin-EDTA at 37°C for 5 minutes. The dental pulp cells detached from the dish are washed, if necessary, then inoculated to a dish for adhesion cell culture, and expansion-cultured. The cells, when reaching subconfluency (state in which the cells occupy approximately 70% of the surface of the culture container) or confluency in macroscopic observation, are detached and recovered from the culture container and inoculated again to a culture container filled with a culture medium. Subculture may be repetitively performed. For example, the cells are allowed to proliferate into a necessary number of cells (e.g., approximately 1 × 10⁷ cells/ml) by performing subculture 1 to 8 times. The detachment of the cells from the culture container can be carried out by a routine method such as trypsin treatment. The cells thus cultured may be recovered and preserved (preservation conditions involve, for example, -150°C).

For example, a basal medium or a basal medium supplemented with serum or the like can be used as the culture solution. DMEM as well as Iscove's modified Dulbecco's medium (IMDM) (Gibco, etc.), Ham's F12 medium (HamF12) (Sigma-Aldrich Co., LLC, Gibco, etc.), RPMI1640 medium, or the like can be used as the basal medium. Two or more basal media may be used in combination. One example of the mixed medium can include a medium of IMDM and HamF12 mixed in equal amounts (e.g., commercially available under a trade name of IMDM/HamF12 (Gibco)). Examples of the component that may be added to the medium can include serum (fetal bovine serum, human serum, sheep serum, etc.), serum replacements (knockout serum replacement (KSR), etc.), bovine serum albumin (BSA), antibiotics, various vitamins, and various minerals.

### (4) Recovery of cell

The cells can be recovered by detaching the cells from the culture container by trypsin treatment or the like, followed by centrifugation. The prevention or treatment agent of the present invention can be prepared using the dental pulp stem cells thus recovered.

In an embodiment, the dental pulp stem cells are a non-transgenic stem cell population derived from human deciduous dental pulp, at least 90% or more of which is CD117-negative, CD73-positive, CD90-positive, and CD105-positive (hereinafter, also referred to as the "stem cell population of the present invention"). Hereinafter, structural or functional characteristics of the stem cell population of the present invention will be described.

### 1.2 Marker

"CD117" is a transmembrane protein that functions as tyrosine kinase receptor and is also called c-kit. Previously known dental pulp stem cells are CD117-positive or are a heterogeneous population comprising CD117-positive cells and CD117-negative cells (Hilkens et al., Cell and Tissue Research (2013) 353, 65-78; Deng et al., Frontiers in Cell and Developmental Biology, March 2021, volume 9, Article 661116; Ferro et al., PLoS ONE July 2012, volume 7, Issue 7, e41774; and Lei et al., Stem Cell International, Volume 2021, Article ID 8886854). By contrast, the stem cell population of the present invention is characterized by being CD117-negative. Also, the stem cell population of the present invention is characterized by being positive for mesenchymal stem cell markers CD73, CD90, and CD105.

"CD325", "CD49d", and "CD51" are cell surface proteins that function as adhesion factors and are also called N-cadherin (cadherin 2), integrin α4, and integrin αV, respectively. Previously known dental pulp stem cells are CD325-positive or are a heterologous population comprising CD325-positive cells and CD325-negative cells (Deng et al., Frontiers in Cell and Developmental Biology, March 2021, volume 9, Article 661116; and Madhoun et al., Frontiers in Cell and Developmental Biology, October 2021, volume 9, Article 717624). By contrast, the stem cell population of the present invention is preferably CD325-negative. Previously known dental pulp stem cells are a heterologous population comprising both cells positive and negative for CD51 and CD49d (Lei et al., Stem Cell International, Volume 2021, Article ID 8886854; and Alvarez et al., International Journal of Oral Science (2015), 7, 205-212). By contrast, the stem cell population of the present invention preferably exhibits a CD49d- and/or CD51-positive ratio of 90%, 95%, 97%, 98%, or 99% or more.

The stem cell population of the present invention is preferably negative for an endothelial cell marker CD31 and also negative for hematopoietic stem cell markers such as CD34-negative and CD45-negative. The stem cell population of the present invention has a positive ratio of 90% or more, preferably 95% or more, for CD150, a positive marker of mesenchymal stem cells, and is preferably negative for negative markers CD14 and CD19.

The stem cell population of the present invention is preferably negative for HLA-DR, HLA-DQ, CD40, CD80, and CD86 related to immunogenicity.

The stem cell population of the present invention is preferably positive for adhesion factors CD29 (ITGB1), CD44, and CD166 (ALCAM) and negative for CD106 (VCAM).

The stem cell population of the present invention is CD73-positive, CD90-positive, and CD105-positive, preferably is CD73-positive, CD90-positive, and CD105-positive and is CD117-negative, further preferably is CD73-positive, CD90-positive, and CD105-positive and is CD117-negative and CD325-negative.

The term "positive" for a marker protein or a marker gene, as used herein, means that the stem cell population exhibits a positive ratio of 30% or more, preferably 40% or more, 50% or more, 60% or more, 70% or more, or 80% or more, more preferably 90% or more, for the protein or the gene when the stem cell population at passages 2 to 8 is assayed by an approach known in the art (e.g., detection data from flow cytometry is analyzed with an isotype control). Further preferably, the term "positive" for a marker protein or a marker gene means that the stem cell population at passages 2 to 8 has a positive ratio of 91%, 92%, 93%, 94%, 95%, 96%, 97%, 98%, or 99% or more for the protein or the gene. Particularly preferably, the term "positive" to a marker protein or a marker gene means that the stem cell population at passages 2 to 8 has a positive ratio of 99.1% or more, 99.2% or more, 99.3% or more, 99.4% or more, 99.5% or more, 99.6% or more, 99.7% or more, 99.8% or more, 99.9% or more, or 100% to the protein or the gene. The term "negative" for a marker protein or a marker gene means that the stem cell population has a positive ratio of less than 10%, preferably less than 7%, more preferably less than 6% or less than 5%, further preferably less than 3% or less than 2%, for the protein or the gene when detected in the same manner as above (however, the value of negativity does not fall below 1% when fluorescence intensity of 1% in an isotype control is set as a positive-negative threshold). In this context, the passage of the cells is performed in a 60% confluent state for adhesion culture.

The cell population of the present invention has the following features:
(1) having a CD117-positive ratio of preferably less than 3%, more preferably less than 2%;
(2) having a CD73-positive ratio of preferably 98% or more, more preferably 99% or more;
(3) having a CD90-positive ratio of preferably 98% or more, more preferably 99% or more; and
(4) having a CD105-positive ratio of preferably 98% or more, more preferably 99% or more
at passages 2 to 8.

The cell population of the present invention may further have, in addition to the features (1) to (4), any two or more, three or more, or four or more of the following features (5) to (20) at passages 2 to 8:
(5) having a CD325-positive ratio of preferably less than 7%, more preferably less than 5%,
(6) having a D49d- and/or CD51-positive ratio of preferably 97% or more, more preferably 98% or more, further preferably 99% or more,
(7) having a CD31-positive ratio of preferably less than 3%, more preferably less than 2%,
(8) having a CD34-positive ratio of preferably less than 7%, more preferably less than 5%,
(9) having a CD45-positive ratio of preferably less than 7%, more preferably less than 5%,
(10) having a CD150-positive ratio of preferably 90% or more, more preferably 95% or more,
(11) having a CD14-positive ratio of preferably less than 7%, more preferably less than 5%,
(12) having a CD19-positive ratio of preferably less than 7%, more preferably less than 5%,
(13) having HLA-DR- and HLA-DQ-positive ratios each of preferably less than 7%, more preferably less than 5%,
(14) having a CD40-positive ratio of preferably less than 7%, more preferably less than 5%,
(15) having a CD80-positive ratio of preferably less than 7%, more preferably less than 5%,
(16) having a CD86-positive ratio of preferably less than 7%, more preferably less than 5%,
(17) having a CD29-positive ratio of preferably 96% or more, more preferably 98% or more,
(18) having a CD44-positive ratio of preferably 96% or more, more preferably 98% or more,
(19) having a CD166-positive ratio of preferably 96% or more, more preferably 98% or more, and
(20) having a CD106-positive ratio of preferably less than 7%, more preferably less than 5%.

The detection of a marker protein can be carried out by immunological assay using an antibody, such as ELISA, immunostaining, or flow cytometry. In the case of a protein that is intracellularly expressed without appearing on cell surface, the protein of interest can be detected by expressing a reporter protein together with the protein and detecting the reporter protein. The detection of a marker gene can be carried out by use of a nucleic acid amplification method and/or a nucleic acid detection method, such as RT-PCR, a microarray, or a biochip.

### 1.3 Cytokine production

The stem cell population of the present invention has a high amount of production of a cytokine useful for tissue regeneration and is rich in the cytokines, as compared with conventional dental pulp stem cells prepared using a medium containing FBS. Particularly, the stem cell population of the present invention has the feature that the ratio of the amount of production of a cytokine useful for tissue regeneration to the amount of production of an inflammatory cytokine is high as compared with the conventional dental pulp stem cells.

Examples of the cytokine useful for tissue regeneration can include SCF (stem cell factor), ANGPTL4 (angiopoietin-like 4), BIGH3 (transforming growth factor-beta-induced), brain-derived neurotrophic factor (BDNF), nerve growth factor (NGF), vascular endothelial growth factor (VEGF), stromal cell-derived factor 1 (SDF-1), monocyte chemoattractant protein-1 (MCP-1), and angiopoietin-2. Examples of the inflammatory cytokine can include IL-6 (interleukin-6).

"SCF" (stem cell factor) is a protein that is generated and secreted (in the present specification, collectively referred to as "produced") by mammalian cells, and is known as a nutritional factor that allows hematopoietic stem cells to proliferation in cultured cell experiments. Also, SCF has been reported to act on neurons or vascular endothelial cells expressing its receptor CD117 and thereby promote neuroprotection (Dhandapani et al., J Neurochem. 2005 Oct; 95 (1): 9-19), neurogenesis (Jin et al., J Clin Invest. 2002 Aug; 110 (3): 311-9; and Osada et al., J Neurosurg Spine. 2010 Oct; 13 (4): 516-23), neurite elongation (Hirata et al., Development. 1993 Sep; 119 (1): 49-56), and angiogenesis (Matsui et al., J Biol Chem. 2004 Apr 30; 279 (18): 18600-7; Sun L., Cancer Cell. 2006 Apr; 9 (4): 287-300; and Kim et al., Cardiovasc Res. 2011 Oct 1; 92 (1): 132-40) in cultured cell experiments or animal experiments. These effects are useful in the treatment of not only nerve disease and ischemic disease but various diseases in need of tissue regeneration, such as intestinal disease and bone disease.

"ANGPTL4"(angiopoietin-like 4) is a protein that is generated and secreted by mammalian cells, and has been reported to have an angiogenic effect (Le Jan et al., Am J Pathol. 2003 May; 162 (5): 1521-8; Hermann et al., Clin Immunol. 2005 Apr; 115 (1): 93-101; and Ma et al., Proc Natl Acad Sci U S A. 2010 Aug 10; 107 (32): 14363-8) and an anti-inflammatory effect (Cho et al., JCI Insight. 2019 Aug 22; 4 (16): e125437) in cultured cell experiments or animal experiments. These effects are useful in the treatment of not only ischemic disease, inflammatory disease, and autoimmune disease but various diseases in need of tissue regeneration.

"BIGH3" (transforming growth factor-beta-induced) is a protein that is generated and secreted by mammalian cells, and is also called βig-H3, keratoepithelin, or TGFBI. BIGH3 has been reported to have an angiogenic effect (Aitkenhead et al., Microvasc Res. 2002 Mar; 63 (2): 159-71) and a bone or cartilage degradation suppressive effect (Ruiz et al., Biomaterials. 2020 Jan; 226: 119544) in cultured cell experiments or animal experiments. These effects are considered useful in the treatment of not only ischemic disease and bone or cartilage disease but various diseases in need of tissue regeneration.

"Angiopoietin-2" is a protein that is generated and secreted by mammalian cells, and is known to participate in lymphangiogenesis in lymphatic endothelial cells and vascular remodeling in tissues (Akwii et al., Cells 2019, 8, 471). Angiopoietin-2 is considered useful in the treatment of diseases in need of angiogenesis and tissue regeneration.

"IL-6" is a protein that is generated and secreted by mammalian cells, and is known as an inflammatory cytokine that increases immune response and tissue inflammation. Also, IL-6 is a representative factor of a series of inflammatory cytokine groups called SASP (senescence-associated secretory phenotype) produced by senile cells, and is considered responsible for chronic inflammation associated with senescence (Rolt et al., Biogerontology. 2019 Jun; 20 (3): 359-371; and Di et al., PLoS One. 2014 Nov 24; 9 (11): e113572). Thus, it is not preferred that cells administered for the purpose of treating a disease should produce a large amount of IL-6.

Specifically, the stem cell population of the present invention is capable of producing SCF at a weight ratio of at least 1/20 or more, preferably a weight ratio of at least 1/10 or more, more preferably a weight ratio of 1/10 to 1/2, further preferably a weight ratio of 1/10 to 1/3, more preferably a weight ratio of at least 1/5 or more, further preferably a weight ratio of at least 1/3 or more, relative to IL-6 (however, without exceeding the amount of production of IL-6) at passages 2 to 8. The stem cell population of the present invention is capable of producing at least 0.1 ng, 0.13 ng, or 0.15 ng, preferably at least 0.17 ng, more preferably at least 0.20 ng, of SCF in 48 hours of culture per 1 × 10⁶ cells at passages 2 to 8.

As in previously known dental pulp stem cells, when cells themselves to be administered to a subject are CD117-positive, SCF produced by the administered cells are first taken up into the administered cells themselves via CD117 of the self or other administered cells. This reduces its action efficiency on endogenous cells of a recipient on which SCF is supposed to act. The stem cell population of the present invention has high ability to produce SCF and is CD117-negative, and as such, is capable of exerting an excellent tissue regenerating effect when administered to a subject in need of tissue regeneration.

Specifically, the stem cell population of the present invention has a CD117-positive ratio of preferably less than 3%, more preferably less than 2%; and is capable of producing SCF at a weight ratio of at least 1/20 or more, preferably a weight ratio of at least 1/10 or more, more preferably a weight ratio of at least 1/5 or more, further preferably a weight ratio of at least 1/3 or more, relative to IL-6 (however, without exceeding the amount of production of IL-6) at passages 2 to 8.

The stem cell population of the present invention is capable of producing ANGPTL4 at a weight ratio of at least 3 or more, preferably a weight ratio of at least 5 or more, more preferably a weight ratio of 5 to 20, further preferably a weight ratio of 5 to 15, further preferably a weight ratio of at least 10 or more, particularly preferably a weight ratio of at least 20 or more, relative to IL-6 at passages 2 to 8. The stem cell population of the present invention is capable of producing at least 4 ng, 4.5 ng, or 5.0 ng, preferably at least 6.9 ng, more preferably at least 7.0 ng, of ANGPTL4 in 48 hours of culture per 1 × 10⁶ cells at passages 2 to 8.

The stem cell population of the present invention is capable of producing BIGH3 at a weight ratio of 400 or more, preferably a weight ratio of 450 or more, more preferably a weight ratio of 450 to 1500, further preferably a weight ratio of 450 to 1000, relative to IL-6 at passages 2 to 8. The stem cell population of the present invention is capable of producing at least 400 ng, 450 ng, or 500 ng, preferably at least 550 ng, more preferably at least 600 ng, of BIGH3 in 48 hours per 1 × 10⁶ cells at passages 2 to 8.

In this context, the weight ratio of each liquid factor in a culture supernatant of the stem cell population of the present invention is determined by measuring the amount of each liquid factor contained in the culture supernatant after culture.

The stem cell population of the present invention is
a cell population capable of producing SCF at a weight ratio of at least 1/20 or more, preferably a weight ratio of at least 1/10 or more, more preferably a weight ratio of at least 1/5 or more, further preferably a weight ratio of at least 1/3 or more, relative to IL-6 (however, without exceeding the amount of production of IL-6); producing ANGPTL4 at a weight ratio of at least 3 or more, preferably a weight ratio of at least 5 or more, more preferably a weight ratio of at least 10 or more, further preferably a weight ratio of at least 20 or more, relative to IL-6; and producing BIGH3 at a weight ratio of 400 or more, preferably a weight ratio of 450 or more, more preferably a weight ratio of 450 to 1500, further preferably a weight ratio of 450 to 1000, relative to IL-6 at passages 2 to 8.

The stem cell population of the present invention is capable of producing SCF at a weight ratio of at least 1/10 or more, preferably a weight ratio of at least 1/5 or more, more preferably a weight ratio of at least 1/2 or more, further preferably at a weight ratio of at least 1/3 or more, relative to IL-6 (however, without exceeding the weight of production of IL-6) at passages 2 to 8 when cultured in the presence of a human platelet lysate (hPL) in a medium free of other animal-derived components (particularly, FBS). The stem cell population of the present invention is capable of producing SCF at a weight ratio of at least approximately 3 or more, preferably at least approximately 5 or more, further preferably at least approximately 7 or more, when cultured in the presence of hPL in a medium free of other animal-derived components, as compared with when cultured in a medium containing FBS.

The stem cell population of the present invention is capable of producing ANGPTL4 at a weight ratio of at least approximately 5 or more, preferably a weight ratio of at least approximately 10 or more, further preferably a weight ratio of at least approximately 20 or more, relative to IL-6 at passages 2 to 8 when cultured in the presence of a human platelet lysate (hPL) in a medium free of other animal-derived components (particularly, FBS). The stem cell population of the present invention is capable of producing ANGPTL4 at a weight ratio of at least approximately 3 or more, preferably at least approximately 5 or more, further preferably at least approximately 7 or more, when cultured in the presence of hPL in a medium free of other animal-derived components, as compared with when cultured in a medium containing FBS.

The stem cell population of the present invention is capable of producing VEGF at a weight ratio of at least approximately 2 or more, preferably a weight ratio of at least approximately 5 or more, further preferably a weight ratio of at least approximately 10 or more, relative to IL-6 at passages 2 to 8 when cultured in the presence of a human platelet lysate (hPL) in a medium free of other animal-derived components (particularly, FBS). The stem cell population of the present invention is capable of producing VEGF at a weight ratio of at least approximately 2 or more, preferably at least approximately 3 or more, further preferably at least approximately 5 or more, when cultured in the presence of hPL in a medium free of other animal-derived components, as compared with when cultured in a medium containing FBS.

The stem cell population of the present invention can reduce the amount of IL-6 production when cultured in the presence of hPL in a medium free of other animal-derived components, as compared with when cultured in a medium containing FBS.

The stem cell population of the present invention has high ability to produce SCF, ANGPTL4, and BIGH3 and a low production ratio of IL-6, and as such, is capable of safely exerting a tissue regenerating effect with low risk of inflammatory response.

### 1.4 Ability to differentiate

The stem cell population of the present invention is multipotent and, as shown in Examples mentioned later, has the ability to differentiate into at least adipocytes, osteoblasts, chondrocytes, and neurons. The differentiation into the cells of interest can be induced as disclosed in Examples of the present specification or in accordance with a method known in the art (e.g., Marion et al., Methods Enzymol. (2006); 420: 339-361; and Wang et al., Molecular Medicine Reports (2016); 14: 5551-5555) by culturing the stem cell population of the present invention in the presence of a differentiation inducing factor appropriate for the cells of interest.

### 1.5 Physiological activity

The stem cell population of the present invention produces a high level of a cytokine useful for tissue regeneration and has, *in vivo* or *ex vivo,* physiological activity (function) such as a neural progenitor cell proliferative effect, a neurite elongating effect, a vascular endothelial cell proliferative effect, a vascular endothelial cell recruiting effect, a vascular-like structure constructing effect, and an immunosuppressive effect.

### 2. Method for producing stem cell population of present invention

The stem cell population of the present invention can be produced by enzymatically isolating cells from the human dental pulp, culturing the cells in a medium free of FBS (fetal bovine serum) in the presence of a human platelet lysate(hPL), and obtaining a colony-forming cell population as the stem cell population.

The "dental pulp" used in the present invention may be any dental pulp of a deciduous tooth or a permanent tooth, and dental pulp of an extracted tooth such as a deciduous tooth or a wisdom tooth is preferred because of its easy availability. For the extracted tooth, it is desired to use a tooth within 72 hours after tooth extraction, more preferably within 48 hours after tooth extraction. It is particularly desired to use a human deciduous tooth preferably within 48 hours after tooth extraction. Examples of the method for enzymatically isolating the cell population from the collected dental pulp include a method of separating cells by treatment with an enzyme containing collagenase, preferably collagenase and neutral protease (e.g., dispase or thermolysin), and centrifuging the cells for isolation. For isolating the cell population from the collected dental pulp, it is preferred to perform treatment with a reagent containing no component derived from mammals and bacteria.

The isolated cells are cultured in the presence of a human platelet lysate (hPL) in a medium free of other animal-derived serum components, particularly, FBS. The "human platelet lysate (hPL)" is obtained by lysing platelet extracted from human blood by a freeze/thaw cycle, and abundantly contains various growth factors and cytokines necessary for cell culture. The amount of the human platelet lysate (hPL) added to the medium or the content thereof in the medium is not particularly limited and is approximately 5 to 20%, preferably 5 to 15%, more preferably 7 to 15%, most preferably approximately 10% for primary culture. The same holds true for subculture.

The term "approximately" as used herein refers to a value that varies within plus and minus 15%, 10%, 8%, 6%, 5%, 4%, 3%, 2%, or 1% base on a reference value. The term "approximately" preferably refers to the range of plus and minus 10%, 5%, or 1% base on a reference value.

The "medium" used in the present invention is not particularly limited as long as the medium is free of other animal-derived unpurified components, particularly, FBS. A serum-free medium is preferably used. The "serum-free medium" as used herein is a medium free of unadjusted or unpurified serum. A medium contaminated with a purified blood-derived component or animal tissue-derived factor (growth factor) is also included in the serum-free medium as long as it does not impairthe object of the present invention. Examples of the serum-free medium can include basal media supplemented with no serum: for example, αMEM medium, DMEM medium, BME medium, Eagle MEM medium, BGJb medium, CMRL 1066 medium, Glasgow MEM (GMEM) medium, Improved MEM Zinc Option medium, IMDM medium, Medium 199 medium, F-12 medium, DMEM/F12 medium, IMDM/F12 medium, Ham's medium, RPMI 1640 medium, Fischer's medium, and mixed media thereof; and commercially available serum-free media for mammalian cells: for example, MesenCult-ACF (STEMCELL Technologies Inc.), STEMPRO MSC SFM (Thermo Fischer Scientific Inc.), and UltraCULTURE Serum-free (Lonza).

The "medium" for clinical application preferably contains no mammal-derived component and is particularly preferably animal-free.

The method for producing the stem cell population of the present invention is preferably a method using a human deciduous tooth within 48 hours after tooth extraction and comprising the steps of: i) enzymatically isolating a cell population from collected dental pulp using a reagent containing no component derived from mammals or bacteria; ii) primary-culturing the isolated cell population in the presence of a human platelet lysate (hPL) in a serum-free medium containing no other animal-derived unpurified components (particularly, FBS) to form colonies; and iii) culturing the obtained colonies in the presence of hPL in a serum-free medium containing no other animal-derived unpurified components (particularly, FBS).

The "medium" may be appropriately supplemented with various nutrient sources necessary for the maintenance and proliferation of the cells or each component necessary for the induction of differentiation without impairing the object of the present invention. Examples of the nutrient source can include carbon sources such as glycerol, glucose, fructose, sucrose, lactose, honey, starch, and dextrin, hydrocarbons such as fatty acids, fat or oil, lecithin, and alcohols, nitrogen sources such as ammonium sulfate, ammonium nitrate, ammonium chloride, urea, and sodium nitrate, inorganic salts such as common salt, potassium salt, phosphate, magnesium salt, calcium salt, iron salt, and manganese salt, monopotassium phosphate, dipotassium phosphate, magnesium sulfate, sodium chloride, ferrous sulfate, sodium molybdate, sodium tungstate, manganese sulfate, various vitamins, and amino acids.

The pH of the medium obtained by blending the components described above is in the range of 6.0 to 9.0, preferably 6.5 to 8.5, more preferably 7.0 to 8.0.

The cells isolated from the dental pulp, and the stem cell population of the present invention are adhesion-cultured. A container is not particularly limited as long as the container can be used for cell culture. A flask, a flask for tissue culture, a dish, a petri dish, a dish for tissue culture, a multidish, a microplate, a microwell plate, a multiplate, a multiwell plate, a microslide, a chamber slide, a petri dish, a tube, a tray, a culture bag, and a roller bottle can be used.

The inoculation density of the cells is not particularly limited and is preferably not too high. For example, cells extracted from the dental pulp of one deciduous tooth is inoculated at an area of 25 to 225 cm², preferably 75 to 150 cm². The culture is carried out at 36°C to 38°C, preferably 36.5°C to 37.5°C, under conditions of 1% to 25% O₂ and 1% to 15% CO₂ with the medium replaced.

The primary culture is preferably performed until colonies are formed and grow and detachment is confirmed. After colony detachment is confirmed, the cells are recovered using a filter or the like. The recovered cells are obtained as a highly homogeneous stem cell population without any special separation approach.

The stem cell population may be further subcultured (expansion-cultured), if necessary. The subculture is carried out using the same medium as that for primary culture. The passage is performed when 60% confluency is attained during adhesion culture. Typically, for the primarily cultured cells, the culture is performed for at least 7 days or longer, preferably 9 days or longer, more preferably 9 to 15 days, in order to confirm colony formation, growth, and detachment, whereas the cells thus passaged can be cultured for at least 1 day or longer, preferably 2 days or longer, more preferably 2 to 3 days. The inoculation density of the cells thus passaged is not particularly limited and is preferably not too high. The cells are inoculated at a density of, for example, 1000 to 2000 cells/cm², preferably 1300 to 1500 cells/cm².

The recovered stem cell population or a culture supernatant thereof is desirably subjected to an endotoxin test, a mycoplasma test, a sterilization test, or the like again for safety.

The produced stem cell population may be cryopreserved (e.g., preserved in a deep freezer of 152°C), if necessary, until use. The cryopreservation is performed, for example, by adding an appropriate cryoprotectant to the medium used in the cell culture. Dextran, DMSO, a commercially available cryopreservation solution, or the like can be used as the cryoprotectant.

### 3. Prevention or treatment of cerebral palsy

The dental pulp stem cells have a prophylactic or therapeutic effect on cerebral palsy and a promoting effect on neurogenesis in the brain. Hence, the dental pulp stem cells can be used as an active ingredient in an agent for preventing or treating cerebral palsy or an agent for promoting neurogenesis. The dental pulp stem cells used in the present invention may be used as, for example, a medicament. The dental pulp stem cells may be used together with an additional active ingredient, and preferably, the dental pulp stem cells are used as a single active ingredient.

Preferably, the dental pulp stem cells used in the present invention are cells that have not undergone induction of differentiation, particularly preferably cells that have not undergone induction of differentiation into neurons. The dental pulp stem cells can be genetically engineered by various known methods. The dental pulp stem cells used in the present invention are preferably non-transgenic cells.

Cerebral palsy is lifelong and variable abnormality of movement, posture, or memory learning in children after birth based on a nonprogressive lesion including neuronal death in the children's brain caused from conception to the neonatal stage (within 4 weeks after birth). However, progressive disease, transient dyskinesia, or motor retardation that seems to return normal in the future is excluded therefrom. Although symptoms or severity of the disorder differs among individuals depending on a lesion site in the brain, there exists classification such as limb rigidity (spastic type), excessive limb movement (athetosic type), and decreased muscle tonus (dyskinetic type). Mental retardation, epilepsy, indirect contracture, and scoliosis are known as complications.

Cerebral palsy is caused by brain malformation or prenatal infection before birth, hypoglycemia, intracranial bleeding, premature birth, or neonatal asphyxia (HIE) at the perinatal stage, and infection of encephalitis, meningitis, or the like for newborns. Among others, cerebral palsy is often caused by perinatal disorder (perinatal cerebral damage).

The type of the cerebral palsy is not particularly limited. Cerebral palsy ascribable to various causes is to be prevented or treated. The dental pulp stem cells are preferably effective for preventing the manifestation of cerebral palsy symptoms caused by perinatal cerebral damage, particularly, symptoms of cerebral palsy caused by hypoxia and/or ischemia, and for treating these symptoms. The treatment may be provided, for example, when a perinatal infant has undergone neonatal asphyxia and/or been diagnosed with periventricular leukomalacia, or may be provided at the chronic stage (6 months or later after birth), and is particularly preferably provided to chronic stage cerebral palsy. The prevention or treatment agent of the present invention may be prophylactically used for, for example, a subject suspected of having cerebral palsy (e.g., a subject who has undergone neonatal asphyxia and/or been diagnosed with periventricular leukomalacia at the perinatal stage), or may be used for suppressing or improving the progression of symptoms in a subject with cerebral palsy symptoms. The prevention or treatment agent of the present invention can be preferably used for improving a motor function and/or a memory learning function in a subject with cerebral palsy symptoms and/or for promoting neurogenesis in the brain of the subject. More preferably, the prevention or treatment agent of the present invention can be used for improving a motor function and a memory learning function in a subject with cerebral palsy symptoms. Further preferably, the prevention or treatment agent of the present invention can be used for improving a motor function and a learning function in a subject with cerebral palsy symptoms and for promoting neurogenesis in the brain of the subject.

Neurogenesis is a phenomenon in which neurons are newly produced from neural stem cells. Neurogenesis is known to occur in particular regions such as the hippocampal dentate gyrus or the subventricular zone not only at the stage of mammalian development but in adults (Gage, J. of Neuroscience, February 1, 2002, 22 (3): 612-613). It is known that when cerebral damage such as perinatal damage occurs, neural stem cells of the subventricular zone or the hippocampal dentate gyrus proliferate and lead to a compensation mechanism in which some of the cells migrate to the brain lesion, whereas the proliferation or migration of such neural stem cells is reduced after a lapse of a given time (approximately 1 month on average in the observation of animal models) after damage (Visco et al., Experimental Neurology 340 (2021) 113643). Hence, the cerebral damage of cerebral palsy cannot be repaired by only the compensation mechanism possessed by organisms as mentioned above (Visco et al., Experimental Neurology 340 (2021) 113643, Chen et al., Frontiers in Pediatrics (2022) 10: 986452).

In the present application, the phrase "promote neurogenesis" by the prevention or treatment of the present invention means that the proliferation of neural stem cells and/or increase in the number of neurons in a brain tissue (particularly, the subventricular zone or the hippocampal dentate gyrus as mentioned above) in a subject with cerebral palsy is markedly enhanced as compared with the case where the prevention or treatment agent of the present invention is not administered.

Neurogenesis in the brain can be detected by combining markers such as bromodeoxyuridine (BrdU), doublecortin (DCX), and Ki-67. NeuN can detect mature neurons in brain tissues.

The prevention or treatment agent of the present invention may be administered to, for example, a subject suspected of having cerebral palsy without manifesting symptoms of cerebral palsy, may be administered to a subject with symptoms of cerebral palsy, or may be administered to a subject diagnosed with cerebral palsy. Examples of the subject suspected of having cerebral palsy include subjects who have undergone a hypoxic state at the perinatal stage, subjects who have undergone ischemia, subjects who have undergone neonatal asphyxia, and subjects who have been diagnosed with periventricular leukomalacia. In the present application, the "subject with symptoms of cerebral palsy" is a subject who manifests one or more symptoms of cerebral palsy and may have already been diagnosed with cerebral palsy or may be before diagnosis.

The prevention or treatment agent of the present invention is not particularly limited as long as the prevention or treatment agent contains the active ingredient. The prevention or treatment agent of the present invention may optionally further contain an additional component. The additional component is not particularly limited as long as the component is pharmaceutically acceptable. The additional component includes a component having a pharmacological effect as well as an additive. Examples of the additional component include carriers or vehicles, excipients, disintegrants, buffers, emulsifiers, suspending agents, soothing agents, stabilizers, preservatives, antiseptics, physiological saline, albumin, media, antibiotics, pH adjusters, growth factors, and hormones. Examples of the carriers or vehicles include physiological saline, Ringer's solutions, and appropriate buffer solutions (e.g., phosphate buffer solutions).

Examples of the subject to which the prevention or treatment agent of the present invention is applied include, but are not particularly limited to, mammals, for example, humans, monkeys, mice, rats, dogs, cats, rabbits, pigs, horses, bovines, sheep, goats, and deers.

The subject to which the prevention or treatment agent of the present invention is applied is preferably a human. In the case of a human, the prevention or treatment agent of the present invention can be used so that the agent is administered from infancy onward (1 year old or later, for example, 2 years old or later, 3 years old or later, 4 years old or later, 5 years old or later, or 6 years old or later). The subject to which the prevention or treatment agent of the present invention is applied can be a subject with symptoms of cerebral palsy, for example, a subject diagnosed as having symptoms of cerebral palsy. The prevention or treatment agent of the present invention can exert a therapeutic effect or the like on cerebral palsy even if administered to the subject.

Examples of the administration route of the prevention or treatment agent of the present invention include intravenous injection (including intravenous drip), intra-arterial injection, intraportal injection, intradermal injection, subcutaneous injection, intramuscular injection, intraperitoneal injection, and transnasal administration. The prevention or treatment agent of the present invention may be locally administered instead of systemic administration. Examples of the local administration can include direct injection to brain tissues. In the case of administering the prevention or treatment agent of the present invention as an injection, the dental pulp stem cells can be suspended, for administration, in a cell culture medium, physiological saline, a composite electrolyte liquid, or the like. Such a suspension may contain serum, albumin, dextran, dimethyl sulfoxide, a cryopreservative solution, or the like.

The content of the dental pulp stem cells in the prevention or treatment agent of the present invention and the dosing schedule thereof can be designed in consideration of the sex, age, body weight, pathological condition, etc. of a subject (patient). The content of the dental pulp stem cells can be, for example, 1 × 10⁵ to 1 × 10¹¹ cells/kg body weight per dose. Single-dose administration as well as continuous or periodic multiple-dose administration may be performed. An appropriate number of doses is 2 to 20 doses (preferably 2 to 10 doses or 2 to 5 doses). The dosing interval can be selected from 1 day to 4 weeks and is preferably 7 days to 24 days or 10 days to 18 days. The multiple-dose administration can more markedly improve a motor function and/or a memory learning function in a subject with cerebral palsy symptoms and/or markedly promote neurogenesis in the brain thereof.

### [Examples]

Hereinafter, the present invention will be described in more detail with reference to Examples. However, the present invention is not limited by these Examples.

### Test Example 1. Preparation of human deciduous dental pulp stem cell

Deciduous dental pulp stem cells were prepared from a human deciduous tooth that fell out. Specifically, the preparation was performed as follows: dental pulp was scraped out of the human deciduous tooth, chopped, and then treated with Liberase (Roche) added at a final concentration of 0.05 mg/ml with stirring at 37°C for 15 minutes. A reaction supernatant containing cells was recovered, and the remaining dental pulp tissue was further treated with Liberase added at 0.05 mg/ml with stirring at 37°C for 15 minutes. Cells were recovered from the treated dental pulp tissue through a 70 mm strainer, combined with the supernatant recovered first, and centrifuged. The recovered cells were inoculated to a culture container using MEMα (Gibco) containing 10% hPL, and isolated and cultured for 9 days or longer until colonies were formed, grew, and were detached. Medium replacement was performed 2 days and 9 days after inoculation. After colony detachment was confirmed, cells were recovered using TrypLE Select (Gibco) and expansion-cultured using the same medium as above.

### Test Example 2. Evaluation of therapeutic effect of deciduous dental pulp stem cell on cerebral palsy

### <Test Example 2-1. Preparation of cerebral palsy model rat>

HIE models (Rice-Vannucci models) were prepared. Specifically, left common carotid artery ligation and hypoxic loading (O₂ 8% for 60 minutes) were performed for 7-day-old male Wistar/ST rats. At the same time therewith, rats of a sham operation group (Sham group) were also prepared.

### <Test Example 2-2. Grouping by behavioral test>

The model rats prepared in Test Example 2-1 were subjected to a horizontal ladder test at the age of 25 to 28 days. Specifically, each rat was allowed to walk on a horizontal ladder, and the gait of a paralyzed leg was scored with 1 to 4 points (0: normal walking, 1: the paralyzed leg slips on the ladder but does not fall out, 2: the paralyzed leg falls out of the ladder, though the next step is normal, 3: the paralyzed leg falls out of the ladder, and the next step is not normal, 4: both legs fall out of the ladder). A score per step (score/step) was calculated. Only individuals given significantly abnormal findings as compared with the Sham group were selected. The selected model rats were divided into two groups (cell administration (Cell) group and a vehicle (Vehicle) group) such that the scores of the test were at the same level between the groups.

### <Test Example 2-3. Administration of cell>

For the Cell group, 1 × 10⁶ ells of the deciduous dental pulp stem cells (Test Example 1) were administered to the tail vein at the age of 35 days (single-dose administration). In the test of another batch, for the Cell group, 1 × 10⁶ cells of the deciduous dental pulp stem cells (Test Example 1) were administered to the tail vein at the ages of 35 days, 49 days, and 63 days (multiple-dose administration). On the other hand, for the Vehicle group, only a medium containing no deciduous dental pulp stem cells was administered in the same manner as in the Cell group.

### <Test Example 2-4. Evaluation test>

The Cell group, the Vehicle group, and the Sham group were subjected to a horizontal ladder test, a cylinder test, and shuttle avoidance.

The horizontal ladder test was conducted by the same method as in Test Example 2-2.

In the cylinder test, each rat was placed in a cylinder. Anterior limb [left (healthy side), right (affected side), or both sides] contacts of the rat to the wall were recorded while rearing to evaluate a use rate of the limb on the affected side. Scores were calculated according to the expression: (L - R) / (L + R + B) (L: left, R: right, B: both). Stronger damage decreases the rate of use of the right (affected side) limb (elevates the score).

In the shuttle avoidance, each rat was placed in a cage, warned by light and sound, and electrically stimulated if there was no avoidance. This operation was repeated to evaluate memory learning capability. 10 × 6 sets per day (a total of 60 sets) were tested for 2 consecutive days, and an avoidance rate for each set was evaluated.

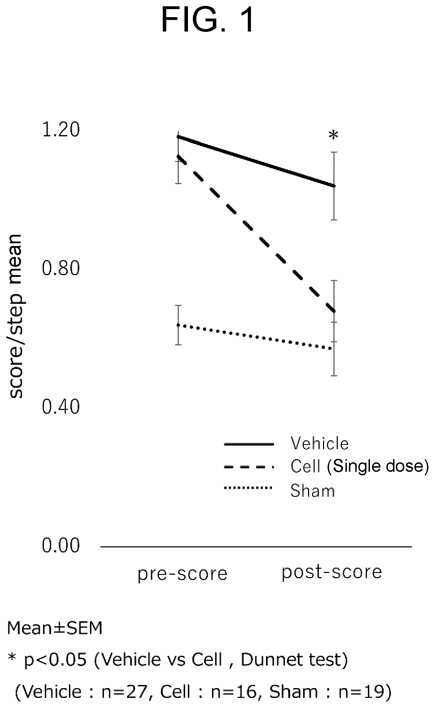

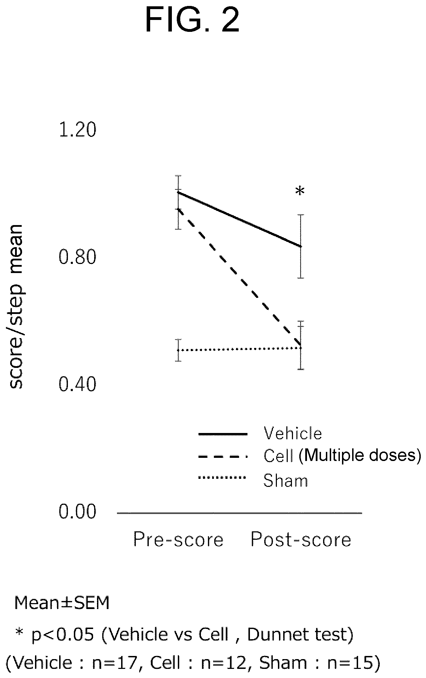

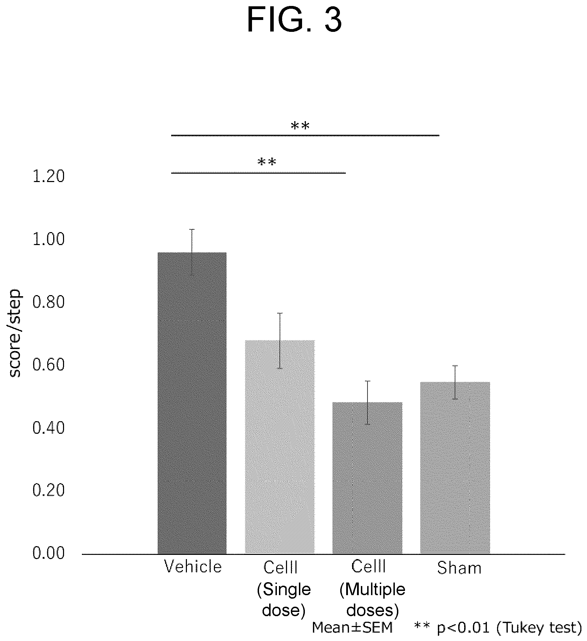

The results of the horizontal ladder test are shown in Figures 1 to 3. The administration of the deciduous dental pulp stem cells was found to improve a motor function evaluated by the horizontal ladder test. The multiple-dose administration tended to exert a stronger improving effect.

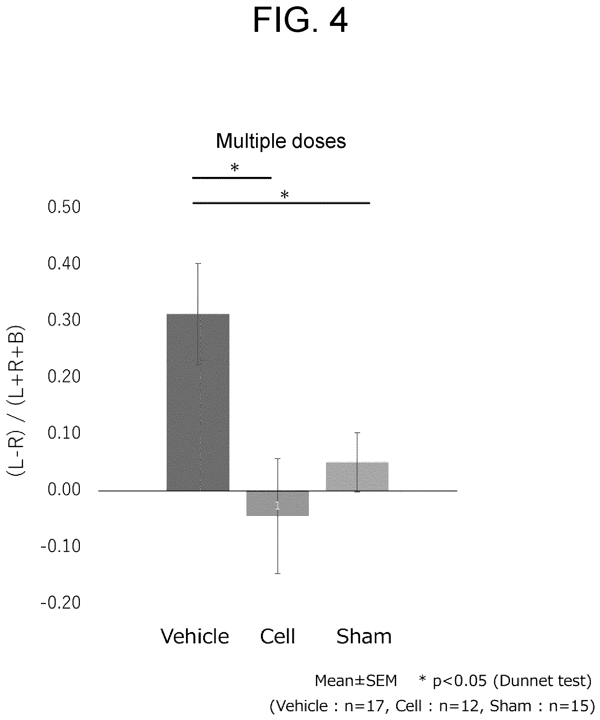

The results of the cylinder test are shown in Figure 4. The administration of the deciduous dental pulp stem cells was found to improve a motor function evaluated by the cylinder test. The multiple-dose administration exhibited a more marked improving effect than that of the single-dose administration.

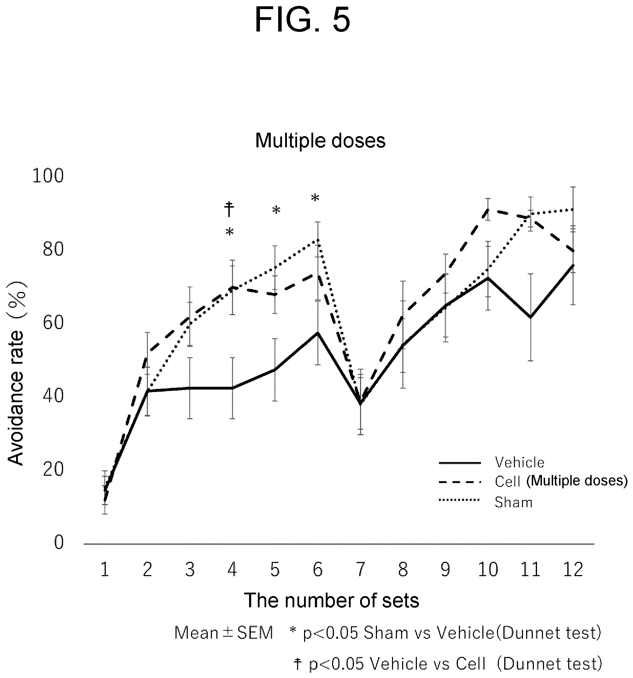

The results of the shuttle avoidance are shown in Figure 5. The administration of the deciduous dental pulp stem cells was found to improve a memory learning function evaluated by the shuttle avoidance. The multiple-dose administration exhibited a more marked improving effect than that of the single-dose administration.

### Test Example 3. Measurement of body weight and brain weight ratio

Body weights and brain weight ratios (cerebrum weight / cerebellum + brain stem weight) at the completion of the test (the age of 110 days) were measured for each group of Test Example 2.

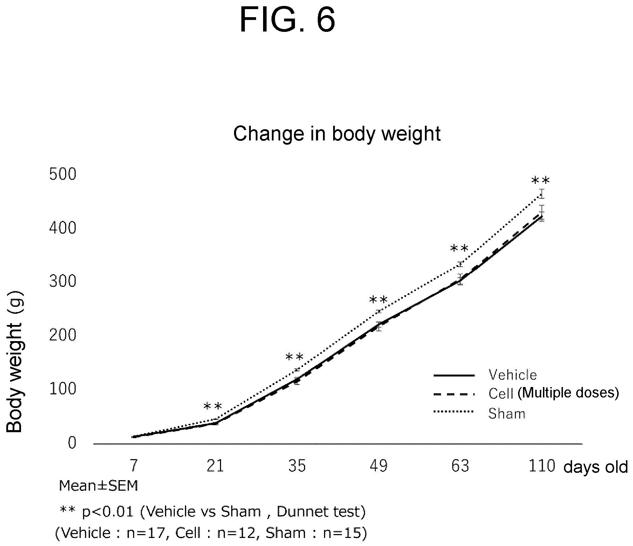

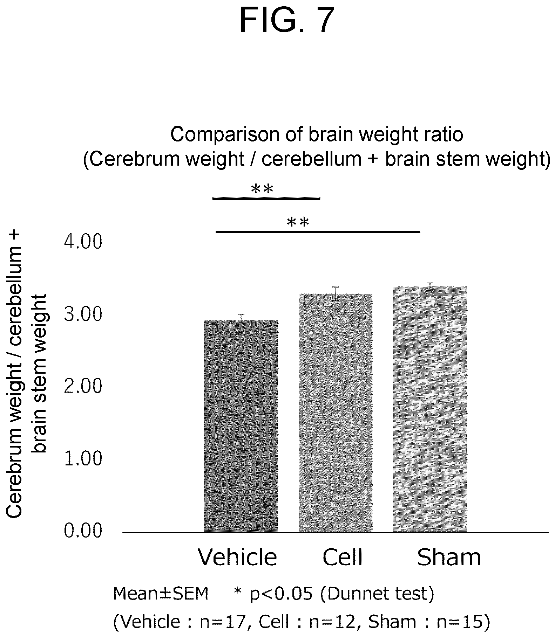

The results of the body weight measurement are shown in Figure 6, and the results of the brain weight ratio measurement are shown in Figure 7. The body weight exhibited a significantly high value in the Sham group and however, did not differ between the Cell group and the Vehicle group. The brain weight ratio (cerebrum weight / cerebellum + brain stem weight) was a significantly low value in the Vehicle group.

### Test Example 4. Mechanism analysis by comprehensive evaluation of target protein

The deciduous dental pulp stem cells were administered in multiple doses in the same manner as in the multiple-dose administration of Test Example 2. The brain was homogenized 2 weeks after final administration of the cells, and extracted proteins were comprehensively analyzed by liquid chromatography-mass spectrometry (LC/MS). Of all the analyzed proteins (n = 1696), proteins detected at a level below detection sensitivity in 5 or more out of six samples in the Vehicle group or the Cell group were excluded (n = 204). 61 types of proteins found to vary significantly in Vehicle group vs Cell group were obtained (p-value < 0.05 (student's t-test) FDR < 0.1 (Storey method)). These are a group of proteins presumably influenced by the administration of the deciduous dental pulp stem cells except for the influence of HIE. As a result of extracting clusters with enrichment score > 2 by GO analysis using DAVID, proteins involved in neurogenesis, morphological formation of cells, axonal new formation, and phosphorylation processes were enriched.

### Test Example 5. In vivo kinetic evaluation of deciduous dental pulp stem cell using quantum dot

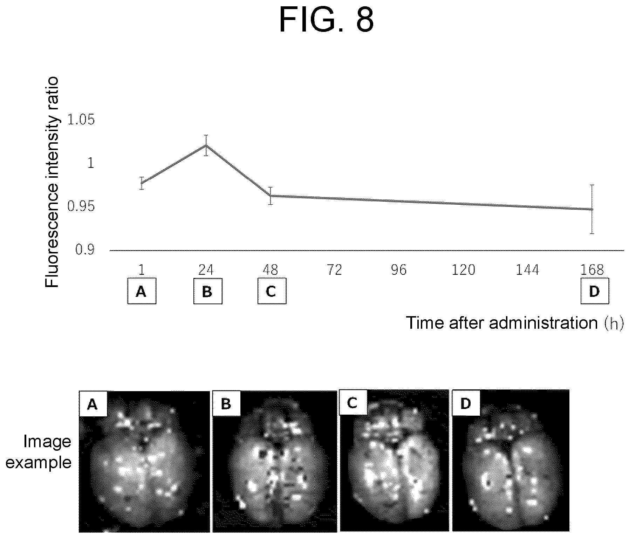

Quantum dot-labeled deciduous dental pulp stem cells (1 × 10⁶ cells/body) were administered to the tail vein of each rat. Evaluation was conducted at four time points (A: 1 hour after injection, B: 1 day, C: 2 days, D: 1 week) after intravenous injection of the cells. The target organ (brain) was taken out at each time point and evaluated for fluorescence intensity using an *in vivo* imaging system. Fluorescence intensity ratio = fluorescence intensity of each individual in the Cell group / mean fluorescence intensity of the Vehicle group at each time point was calculated on the basis of the fluorescence intensity. The results are shown in Figure 8.

### Test Example 6. Evaluation of neurogenetic effect using deciduous dental pulp stem cells

### <Test Example 6-1: Neurogenesis marker>

The deciduous dental pulp stem cells were administered in multiple doses to each HIE model (Rice-Vannucci model) rat in the same manner as in Test Examples 2-1 to 2-3. Bromodeoxyuridine (BrdU) at 50 mg/kg was intraperitoneally administered once a day for a total of 3 days from the day following the third administration of the cells.

At 2 weeks after the third administration of the cells, the rat was perfusion-fixed in paraformaldehyde (PFA), and a brain tissue was collected and embedded in a paraffin block. This block was sliced to prepare brain tissue slices, followed by (1) immunohistological staining using an anti-BrdU antibody and (2) fluorescent double immunostaining with an anti-BrdU antibody and an anti-doublecortin (DCX) antibody.

The hippocampal dentate gyrus and the subventricular zone on the damage side were evaluated for each slice, and the number of BrdU-positive cells and the ratio of BrdU/DCX double stain-positive cells to the BrdU-positive cells were calculated.

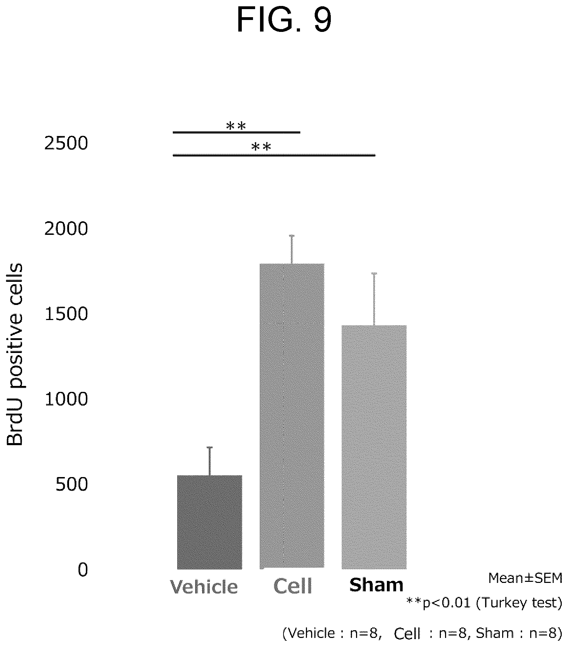

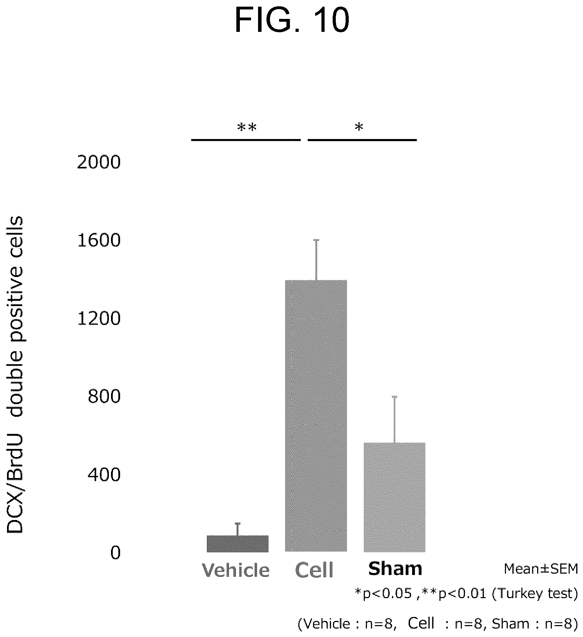

As a result, in the immunohistological staining with an anti-BrdU antibody, BrdU-positive cells were found in the innermost layer of the hippocampal dentate gyrus on the damage side, the subependymal layer of the lateral ventricle, and the corpus striatum in the Cell group. In the fluorescent double staining, the ratio of BrdU/DCX double stain-positive cells in the hippocampal dentate gyrus on the damage side was significantly increased in the Cell group (Figure 9). Further, the number of BrdU-positive cells was significantly increased in the Cell group (Figure 10).

### <Test Example 6-2: Neural marker>

Each individual (Vehicle, Cell, and Sham groups) subjected to the behavioral test in Test Example 2 was perfusion-fixed in PFA at the age of 4 months after birth, and a brain tissue was collected and embedded in a paraffin block. This block was sliced to prepare brain tissue slices, followed by immunohistological staining using an anti-NeuN antibody. The number of NeuN-positive cells in the hippocampal dentate gyrus on the damage side was measured in these slices by the stereology method.

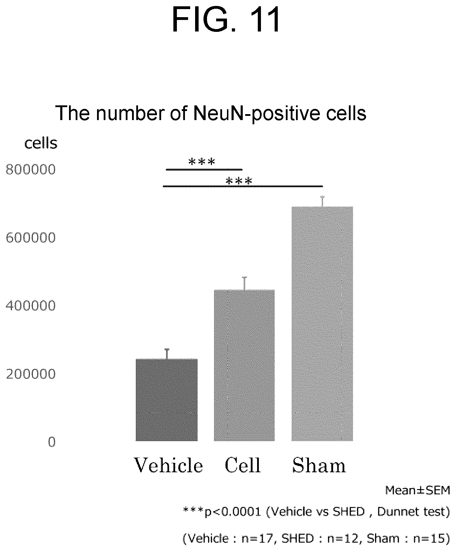

As a result, the number of NeuN-positive cells in the hippocampal dentate gyrus exhibited a significantly high value in the Cell group compared to the Vehicle group (Figure 11).

The results of this test demonstrated that intravenously administered dental pulp stem cells are capable of migrating into the brain of a recipient. The results also demonstrated that the administration of the dental pulp stem cells promotes neurogenesis in the damaged brain, particularly, the hippocampal dentate gyrus.

The test results disclosed in the present invention suggest that neurogenesis is involved in improvement in motor function or memory learning found upon administration of the deciduous dental pulp stem cells to an individual with cerebral palsy.

### Test Example 7. Effect on perinatal hypoxic-ischemic encephalopathy

### <Test Example 7-1: Preparation of dental pulp stem cell>

In a sterile environment, dental pulp was scraped out of an extracted tooth (human deciduous tooth) within 48 hours after tooth extraction, chopped, and then treated with Liberase (MNP-S GMP Grade, Roche) added at a final concentration of 0.05 mg/ml with stirring at 37°C for 15 minutes in the same manner as in Test Examples 1. A reaction supernatant containing cells was recovered, and the remaining dental pulp tissue was further treated with Liberase added at 0.05 mg/ml with stirring at 37°C for 15 minutes. Cells were recovered from the treated dental pulp tissue through a 70 mm strainer, combined with the supernatant recovered first, and centrifuged. The recovered cells were inoculated to a culture container (CellBind T75 flask) using MEMα (Gibco) containing 10% hPL (AventaCell BioMedical Co., Ltd.), and isolated and cultured for 9 days or longer until colonies were formed, grew, and were detached. Medium replacement was performed 2 days and 9 days after inoculation. After colony detachment was confirmed, cells were recovered using TrypLE Select (Gibco) and expansion-cultured using the same medium as above.

### <Test Example 7-2: Evaluation test in cerebral palsy model rat>

The left carotid artery of a 7-day-old Wistar/ST rat was ligated under anesthesia, and the rat was left for 1 to 2 hours and then left for 1 hour in a hypoxic (8% O₂) environment to prepare a newborn rat hypoxic ischemic encephalopathy model. On the next day, a solvent or the human dental pulp stem cells of the present invention at 1 × 10⁵ cells were administered into the vein to study a motor function improving effect on perinatal hypoxic ischemic encephalopathy. Specifically, a rotarod test was conducted 41 days after administration, and limb motor coordination and endurance were evaluated by measuring a latent time to fall from a rod.

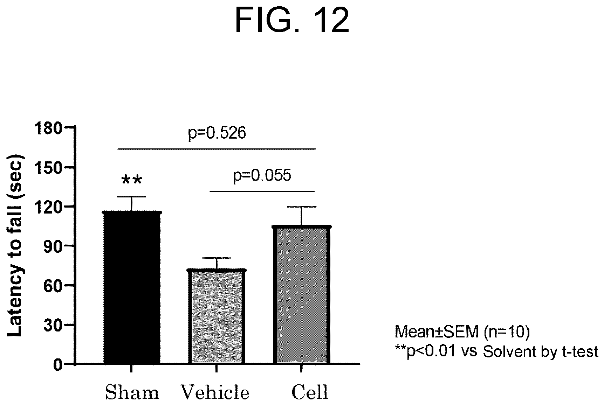

As a result, a drastically shortened latent time to fall, i.e., an obvious disorder in motor function, was found in the solvent administration group (Vehicle) compared with a sham treatment group (normal group, Sham), whereas the latent time to fall was rarely shortened in the cell administration group (Cell), showing a restored motor function (Figure 12). This suggested that the present human dental pulp stem cells are capable of recovering symptoms of cerebral palsy in a relatively short period upon administration using a clinically implementable route and timing in perinatal hypoxic ischemic encephalopathy.

### Test Example 8. Effect on severe perinatal hypoxic ischemic encephalopathy

The right common carotid artery of an SD rat of 7 days after birth was ligated under anesthesia and then cut, and the rat was left for 105 minutes in a hypoxic (8% O₂) environment to prepare a severe hypoxic ischemic encephalopathy model of the newborn rat. At the same time therewith, a sham treatment group was also prepared. At the age of 35 days, grouping was performed by the rotarod method to establish three groups, a sham treatment group (Sham), a solvent administration group (Vehicle), and a cell administration group (Cell). At the ages of 5 weeks, 7 weeks, 9 weeks, and 11 weeks (four doses at 2-week intervals), the human dental pulp stem cells the present invention was administered (1 × 10⁷ cells/kg) into the tail vein. At the age of 13 weeks, a cylinder test was conducted in a blind manner. At the age of 14 weeks, the animals were euthanized, and the whole brain was excised from half the number of animals in each group (5 animals in the sham treatment group and 6 animals each in the solvent administration group and the cell administration group). Then, tissues were fixed in 4% paraformaldehyde-phosphate buffer solution. Cerebrum tissue samples were embedded in paraffine and sliced in accordance with routine methods, and all the samples were stained with HE and immunostained with myelin basic protein (MBP). Since almost all of the individuals in the solvent administration group and the cell administration group lacked a great part of the cerebrum on the disorder side (right side), the total length of MBP was subjected to image analysis in a blind manner for the left cerebrum tissues on the non-disorder side.

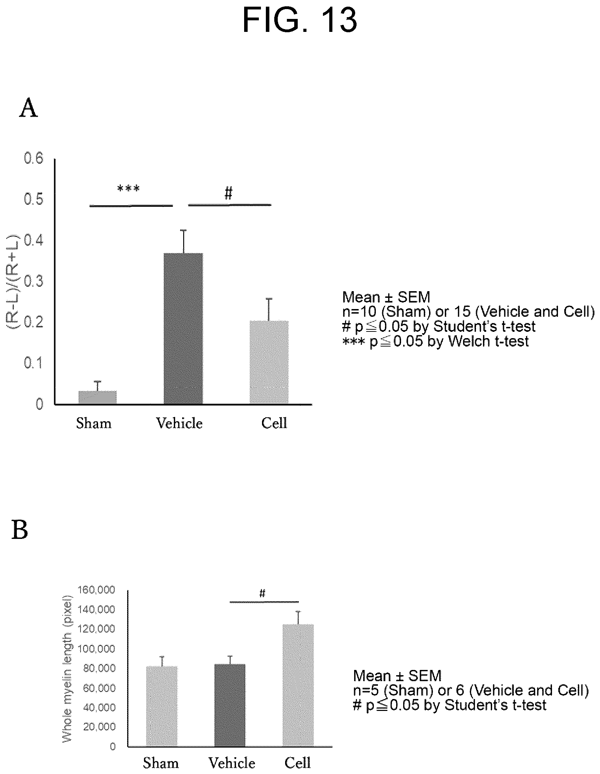

As a result of the cylinder test, the usage rate of the left anterior limb controlled by the brain on the disorder side exhibited a significantly high value in the cell administration group compared with the solvent administration group. Thus, an improving effect of the administration of the cells on motor dysfunction was found (Figure 13A). Immunostaining with MBP was performed for the purpose of studying the relationship between the administration of the cells and histological change in cerebrum tissue, and a nerve fiber length was calculated from a staining image thereof. Significant increase in total nerve fiber length was found in the cell administration group compared with the solvent administration group (Figure 13B). These results suggest the possibility that under the conditions of this test, the present human dental pulp stem cells elongate (or increase) nerve fiber in the cerebrum on the non-disorder side and contribute to functional restoration of the left anterior limb on the disorder side.

These results demonstrated that repetitive administration using a clinically implementable administration route and administration timing in severe perinatal hypoxic ischemic encephalopathy improves motor dysfunction of cerebral palsy and nerve elongation on the non-disorder side contributes to a partial mechanism of action thereof.

### Test Example 9. Surface marker analysis

Human dental pulp stem cells prepared from eight donors prepared from extracted deciduous teeth in the same manner as in Test Example 1 were reacted at 1 × 10⁵ cells per donor with phycoerythrin (PE)-labeled antibodies against various surface markers (CD117 (#313204), CD31 (#303105), CD34 (#343606), CD45 (#368510), CD90 (#328110), CD105 (#323206), CD73 (#344004), CD325 (#350806), CD49d (#304303), CD51 (#327909), CD29 (#303003), CD44 (#338807), CD166 (#343904), and CD106 (#305805), all manufactured by BioLegend, Inc.), and data was obtained by use of flow cytometry. Analysis was conducted by gating such that an isotype control antibody-positive ratio would be around 1%, and by calculating positive ratios for the various surface markers.

**[Table 1]**

| Positive ratio for surface marker (%) | | | | | | | | |
|---|---|---|---|---|---|---|---|---|
| | Donor 1 | Donor 2 | Donor 3 | Donor 4 | Donor 5 | Donor 6 | Donor 7 | Donor 8 |

| | | | | | | | | |
|---|---|---|---|---|---|---|---|---|
| CD117 | 1.99 | 1.82 | 1.36 | 1.36 | 0.58 | 1.10 | 0.91 | 1.20 |
| CD31 | 4.16 | 6.12 | 1.32 | 5.37 | 2.20 | 3.13 | 2.06 | 15.49 |
| CD34 | 1.54 | 1.52 | 0.17 | 0.72 | 6.86 | 19.96 | 1.31 | 3.29 |
| CD45 | 0.97 | 0.92 | 0.22 | 1.36 | 0.46 | 0.90 | 0.56 | 0.51 |
| CD90 | 100.00 | 100.00 | 99.99 | 100.00 | 100.00 | 100.00 | 99.99 | 100.00 |
| CD105 | 100.00 | 99.99 | 100.00 | 100.00 | 100.00 | 100.00 | 100.00 | 100.00 |
| CD73 | 100.00 | 99.99 | 99.99 | 100.00 | 99.99 | 100.00 | 100.00 | 99.99 |
| CD325 | 2.31 | 1.68 | 2.04 | 3.28 | 4.44 | 1.74 | 1.40 | 4.10 |
| CD49d | 96.10 | 99.60 | 99.22 | 99.98 | 99.99 | 99.60 | 99.91 | 99.99 |
| CD51 | 100.00 | 100.00 | 99.99 | 100.00 | 100.00 | 100.00 | 100.00 | 99.99 |

As a result, all the donor cells had a positive ratio of less than 5% for CD117, CD325, and CD45 and were thus determined as being negative therefor. All the donor cells had a positive ratio of 96% or more for CD73, CD90, CD105, CD49d, and CD51 and were thus determined as being positive therefor.

### Test Example 10. Cytokine production

The present human dental pulp stem cells were subcultured in 10% hPL/MEMα or 10% FBS/MEMα for 7 days and then inoculated at 5.76 × 10⁵ cells/2.5 ml/well to a 6-well plate using the same medium as above, followed by replacement with hPL-free, FBS-free, and phenol red-free MEMα at 2.5 ml/well on the next day. A culture supernatant was recovered 48 hours later, dispensed, and stored at -80°C. Frozen samples were thawed. SCF, ANGPTL4, BIGH3, and IL-6 concentrations in the samples were measured using Multiplex HGF panel (BioLegend, Inc., #740180), Human ANGPTL4 assay kit (IBL, #27749), Human beta IG-H3 ELISA (Abcam plc, #ab220651), and Multiplex Neuroinflammatory panel (BioLegend, Inc., #740796), respectively, and their respective concentration ratios to IL-6 were calculated (Tables 2 and 3). From the amount of the culture supernatant and the number of inoculated cells, the amounts of various factors contained in the cell culture solution were calculated as the amounts of production per 1 × 10⁶ cells (Tables 4 and 5).

As a result, the culture supernatant of the dental pulp stem cells subcultured in 10% hPL/MEMα had higher concentration ratios to IL-6 and amounts of production per 1 × 10⁶ cells for all of SCF, ANGPTL4, and BIGH3 than those of the culture supernatant of the dental pulp stem cells subcultured in 10% FBS/MEMα.

**[Table 2]**

| Concentrations and ratios of SCF, ANGPTL4, BIGH3, and IL-6 produced by human dental pulp stem cell cultured in 10% hPL/MEMα | | | | | | |
|---|---|---|---|---|---|---|
| | Concentration in culture supernatant (ng/mL) | | | | Ratio to IL-6 | |
| | Donor 1 | | Donor 2 | | Donor 1 | Donor 2 |
| | n=3 | Mean | n=3 | Mean | Mean | Mean |
| SCF | 0.0406 | 0.0395 | 0.0767 | 0.0606 | 0.29 | 0.21 |
| | 0.0396 | | 0.0598 | | | |
| | 0.0383 | | 0.0452 | | | |
| ANGPTL4 | 1.36 | 1.61 | 2.61 | 3.41 | 12 | 12 |
| | 1.68 | | 3.92 | | | |
| | 1.79 | | 3.69 | | | |
| BIGH3 | 71.5 | 128 | 123 | 140 | 944 | 487 |
| | 166 | | 130 | | | |
| | 147 | | 167 | | | |
| IL-6 | 0.167 | 0.136 | 0.266 | 0.288 | | |
| | 0.122 | | 0.261 | | | |
| | 0.119 | | 0.337 | | | |

**[Table 3]**

| Concentrations and ratios of SCF, ANGPTL4, BIGH3, and IL-6 produced by human dental pulp stem cell cultured in 10% FBS/MEMα | | | | | | |
|---|---|---|---|---|---|---|
| | Concentration in culture supernatant (ng/mL) | | | | Ratio to IL-6 | |
| | Donor 1 | | Donor 2 | | Donor 1 | Donor 2 |
| | n=3 | Mean | n=3 | Mean | Mean | Mean |
| SCF | 0.0125 | 0.0117 | 0.0135 | 0.0145 | 0.05 | 0.04 |
| | 0.0112 | | 0.0153 | | | |
| | 0.0114 | | 0.0149 | | | |
| ANGPTL4 | 0.417 | 0.574 | 0.143 | 0.141 | 2.3 | 0.36 |
| | 0.592 | | 0.143 | | | |
| | 0.712 | | 0.138 | | | |
| BIGH3 | 71.2 | 77.2 | 54.2 | 53.5 | 309 | 135 |
| | 58.3 | | 58.3 | | | |
| | 101.9 | | 48.0 | | | |
| IL-6 | 0.350 | 0.249 | 0.375 | 0.397 | | |
| | 0.196 | | 0.486 | | | |
| | 0.202 | | 0.331 | | | |

**[Table 4]**

| Amounts of SCF, ANGPTL4, BIGH3, and IL-6 produced by human dental pulp-derived cell cultured in 10% hPL/MEMα | | | | |
|---|---|---|---|---|
| | Amount of secretion into culture supernatant (ng/10⁶ cells) | | | |
| | Donor 1 | | Donor 2 | |
| | n=3 | Mean | n=3 | Mean |
| SCF | 0.176 | 0.171 | 0.333 | 0.263 |
| | 0.172 | | 0.260 | |
| | 0.166 | | 0.196 | |
| ANGPTL4 | 5.89 | 6.99 | 11.3 | 14.8 |
| | 7.30 | | 17.0 | |
| | 7.78 | | 16.0 | |
| BIGH3 | 311 | 557 | 535 | 609 |
| | 723 | | 564 | |
| | 639 | | 727 | |
| IL-6 | 0.724 | 0.591 | 1.16 | 1.25 |
| | 0.531 | | 1.13 | |
| | 0.516 | | 1.46 | |

**[Table 5]**

| Amounts of SCF, ANGPTL4, BIGH3, and IL-6 produced by human dental pulp-derived cell cultured in 10% FBS/MEMα | | | | |
|---|---|---|---|---|
| | Amount of secretion into culture supernatant (ng/10⁶ cells) | | | |
| | Donor 1 | | Donor 2 | |
| | n=3 | Mean | n=3 | Mean |
| SCF | 0.0542 | 0.0508 | 0.0584 | 0.0631 |
| | 0.0486 | | 0.0663 | |
| | 0.0496 | | 0.0646 | |
| ANGPTL4 | 1.81 | 2.49 | 0.619 | 0.613 |
| | 2.57 | | 0.619 | |
| | 3.09 | | 0.600 | |
| BIGH3 | 309 | 335 | 235 | 232 |
| | 253 | | 253 | |
| | 442 | | 208 | |
| IL-6 | 1.52 | 1.08 | 1.63 | 1.72 |
| | 0.852 | | 2.11 | |
| | 0.877 | | 1. 44 | |

### Test Example 11. Effect on neurogenesis

### <Test Example 11-1: Coculture experiment with neural stem cell>

Neural stem cells (NSC) (Sigma-Aldrich Co., LLC, SCR022) recovered from the hippocampal dentate gyrus of an adult rat were primarily cultured (medium: 100 ml of DMEM/F-12 (Dulbecco's Modified Eagle Medium/Nutrient Mixture F-12), 2.0 ml of B-27 Supplement (50X) serum free, 1.0 ml of GlutaMAX Supplement, and 20 µl of basic fibroblast growth factor (0.1 mg/ml)) in a 24-well plate in accordance with the attached protocol and used in subsequent experiments.

After a lapse of 24 hours from the primary culture of NSC, human deciduous tooth-derived dental pulp stem cells (SHED) obtained in the same manner as in Test Example 1, human bone marrow-derived mesenchymal stem cells (BM-MSC), or human skin fibroblasts inoculated (2 × 10^4 cells/well for each cell type) to an insert (Corning Inc., Culture Insert, 24-well, 0.4 µm PET transparent, part number: 353095) were placed on each well inoculated with NSC (1 × 10^4 cells/well), and cocultured in a serum-free medium(Gibco, MEM α nucleosides no phenol red INV, part number: 41061029). NSC cultured alone was used as a coculture control.

### <Test Example 11-2: Cell staining>

At 48 hours after the start of coculture, a cell supernatant was recovered, and the cells on the plate were fixed in PFA. These cells were stained with Hoechst (nucleus), an anti-SOX2 antibody (neuron marker), and an anti-S-100 antibody (astrocyte marker). As a result of the staining, the cells were SOX2-positive and however, were rarely S-100-positive. Thus, SHED in the culture for 48 hours was confirmed to have no influence on the induction of differentiation of NSC.

### <Test Example 11-3: Evaluation of cell proliferation rate>

Among the cells stained in Test Example 11-2, SOX2-positive cells were counted by the stereology method and normalized with the number of control cells to calculate a cell proliferation rate (growth rate).

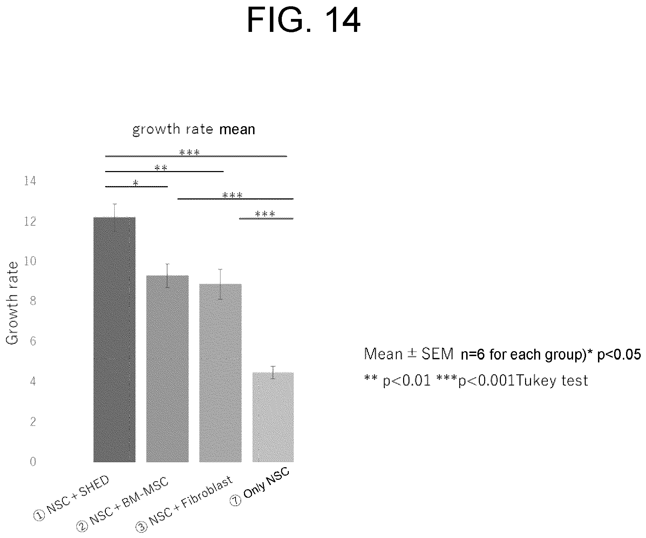

As a result, SHED was found to exhibit the highest proliferative effect on NSC (Figure 14).

### <Test Example 11-4: Evaluation of BCNF concentration in culture supernatant>

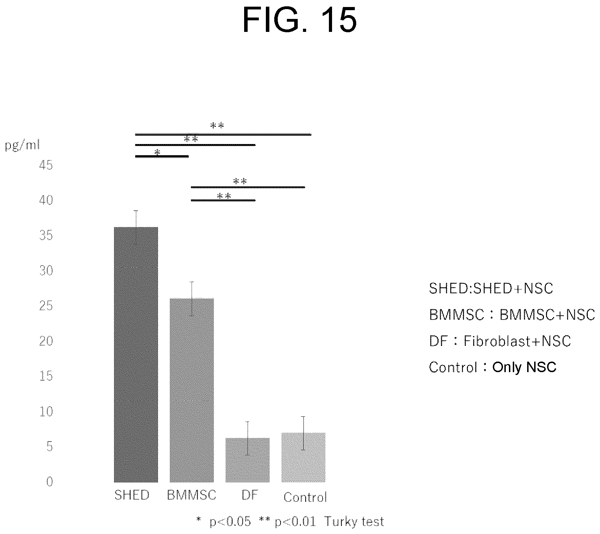

For the coculture performed in Test Example 11-3, the concentration of brain-derived nutritional factor (BDNF) in the culture supernatant on the NSC culture side recovered before cell fixation was measured by ELISA. As a result, the coculture with SHED was found to have a significantly high BDNF concentration in the culture supernatant (Figure 15).

These results indicated that SHED contributes to the proliferation of NSC by coculture with the NCS, and demonstrated that its effect was greater than that of BM-MSC or fibroblasts.

In addition, the culture supernatant of NSC cocultured with SHED was found to be rich in BDNF. "BDNF" (brain-derived neurotrophic factor) is a protein that is generated and secreted by mammalian cells, and is widely distributed in the brain of mammals. BDNF, which is a neurotrophic factor, is known to promote the development and elongation of neurons and participate in the regulation of synaptic functions (Kowianski et al., Cell Mol Neurobiol (2018) 38: 579-593), and has been reported to have a neuroprotective effect (Lau et al., Cell Reports (2015) 12: 1353-1366; and Numakawa et al., Histol Histopathol (2010) 25: 237-258). These effects are considered useful in the treatment of not only neurodegenerative disease but various diseases in need of nerve tissue regeneration.

The results of this test indicate the possibility that SHED contributes to neurogenesis by promoting the proliferation of NSC remaining at a degeneration site of a brain nerve tissue, for example, through the production of BDNF.

All publications, patents, and patent applications cited herein are incorporated herein by reference in their entirety.

## Claims

1. An agent for preventing or treating cerebral palsy, comprising dental pulp stem cells, wherein the prevention or treatment agent is administered to a subject with symptoms of cerebral palsy.

2. The prevention or treatment agent according to claim **1,** wherein the cerebral palsy is chronic stage cerebral palsy.

3. The prevention or treatment agent according to claim 2, wherein the chronic stage is from infancy onward.

4. The prevention or treatment agent according to claim 1, wherein the prevention or treatment agent is administered in multiple doses.

5. The prevention or treatment agent according to claim 1, wherein the dental pulp stem cells are human deciduous dental pulp stem cells.

6. The prevention or treatment agent according to claim 1, wherein the cerebral palsy is cerebral palsy caused by hypoxia and/or ischemia.

7. The prevention or treatment agent according to claim 1, wherein the cerebral palsy is caused by perinatal cerebral damage.

8. The prevention or treatment agent according to claim 1, wherein the prevention or treatment agent is used for improving a motor function and/or a memory learning function of the subject with symptoms of cerebral palsy.

9. The prevention or treatment agent according to claim 1, wherein the subject is a human.

10. The prevention or treatment agent according to claim 1, wherein the prevention or treatment agent causes neurogenesis in the brain of the subject.

11. The prevention or treatment agent according to claim 1, wherein the dental pulp stem cells consist of a stem cell population derived from human deciduous dental pulp, the stem cell population being **characterized in that** 90% or more of the stem cell population is CD117-negative, CD73-positive, CD90-positive, and CD105-positive.

12. The prevention or treatment agent according to claim 11, wherein 90% or more of the stem cell population is CD325-negative or is CD51-positive.

13. The prevention or treatment agent according to claim 11, wherein the stem cell population has at least one of the following characteristics 1) to 3):
1) producing SCF (stem cell factor) at a weight ratio of 1/10 or more relative to IL-6,
2) producing ANGPTL4 (angiopoietin-like 4) at a weight ratio of 5 or more relative to IL-6,
3) producing BIGH3 (transforming growth factor-beta-induced) at a weight ratio of 450 or more relative to IL-6.

14. The prevention or treatment agent according to claim 11, wherein the stem cell population has at least one of the following characteristics 1) to 3):
1) producing at least 0.1 ng of SCF (stem cell factor) in 48 hours per 1 × 10⁶ cells,
2) producing at least 500 ng of BIGH3 (transforming growth factor-beta-induced) in 48 hours per 1 × 10⁶ cells,
3) producing at least 5 ng of ANGPTL4 in 48 hours per 1 × 10⁶ cells.

15. The prevention or treatment agent according to any of claim 11 to 14, wherein the stem cell population is obtained by culturing cells enzymatically isolated from the human dental pulp in a medium free of FBS (fetal bovine serum) in the presence of a human platelet lysate.
